# FASCICULE DE BREVET EUROPEEN

(11) **EP 2 797 517 B1**
(45) Date de publication et mention de la délivrance du brevet: **11.01.2017**
(21) Numéro de dépôt: 12823002.6
(22) Date de dépôt: 17.12.2012
(51) Int. Cl.: G01N 27/62, G01N 33/543, G01N 21/64, A61B 10/00, A61B 10/02

(54) **DISPOSITIF DE PRÉLÈVEMENT ET ANALYSE D'ESPÈCES BIOLOGIQUES OU BIOCHIMIQUES.**
VORRICHTUNG ZUR PROBENNAHME UND ANALYSE VON BIOLOGISCHEN ODER BIOCHEMISCHEN SPEZIES
DEVICE OF SAMPLING AND ANALYSING BIOLOGICAL OR BIOCHEMICAL SPECIES

(30) Priorité: 29.12.2011 FR 1162530
(43) Date de publication de la demande: 05.11.2014
(73) Titulaire: Commissariat à l'Énergie Atomique et aux Énergies Alternatives, 75015 Paris (FR)
(72) Inventeur: BOUAMRANI, Ali, 38000 Grenoble (FR); BERGER, François, F-38240 Meylan (FR); COSNIER, Marie-Line, F-38000 Grenoble (FR)
(74) Mandataire: Gevers & Orès
(86) Numéro de dépôt international: PCT/IB2012/057387
(87) Numéro de publication internationale: WO 2013/098703

(56) Documents cités:
- WO-A1-2006/131400
- WO-A1-2011/025602
- US-A1- 2011 213 270

## Description

L'invention porte sur un dispositif de prélèvement in vivo d'espèces biologiques ou biochimiques.

Une des priorités de la recherche biomédicale est d'améliorer les analyses anatomo-pathologiques, histologiques et moléculaires. L'approche histologique conventionnelle se base sur des techniques de biopsie visant à prélever des échantillons de tissus biologiques. Ces techniques demeurent relativement intrusives, malgré les progrès rendus possibles par la miniaturisation des dispositifs de mise en oeuvre. En outre, les procédures de préparation des tissus (fixation en paraffine, congélation...) sont peu compatibles avec les nouvelles approches d'investigation moléculaire. La fragilité des espèces biochimiques telles que l'ARN et les protéines est l'un des facteurs pré-analytiques qui explique l'échec relatif du transfert clinique des approches « poly-omiques » innovantes.

Par ailleurs les procédures actuelles, en raison de leur coût et du temps requis pour leur mise en oeuvre, sont difficilement compatibles avec le besoin de disposer d'analyses extemporanées, c'est à dire réalisées au bloc opératoire pendant une intervention chirurgicale, afin d'aider les décisions du chirurgien. De plus, l'analyse histologique utilisant des colorations rapides ne permet pas toujours d'obtenir des informations suffisamment pertinentes.

Le document WO 2006/082344 décrit un dispositif de prélèvement moléculaire par contact comprenant un support ayant une face structurée à l'échelle micrométrique, se présentant par exemple sous la forme d'un réseau de micro-plots, ou bien de micro-cuvettes pouvant être remplies de micro-billes. Cette face, qui est de préférence fonctionnalisée, présente une surface développée relativement importante, susceptible de capturer par simple contact et de fixer des molécules d'intérêt contenues dans un tissu biologique. Le recours à une fonctionnalisation de la surface introduit des contraintes au niveau de la conservation du dispositif lors de son stockage et au niveau de la stérilisation (les procédés de stérilisation conventionnels étant susceptibles d'affecter la fonctionnalisation). Et, en l'absence de fonctionnalisation, l'efficacité et la sélectivité de la capture sont insuffisantes.

L'invention vise à surmonter ces inconvénients de l'art antérieur. Conformément à l'invention, ce résultat est atteint grâce à l'utilisation d'une surface de contact nano-poreuse, et en particulier en silicium nano-poreux. Les nano-pores permettent une adsorption efficace et relativement sélective d'espèces chimiques et biologiques, même en l'absence d'une fonctionnalisation de surface (bien qu'une fonctionnalisation puisse être également prévue dans certains modes de réalisation, pour améliorer encore la sélectivité et/ou l'efficacité de la capture) ; cela est attribué à un phénomène de succion par les pores. Le document WO 2011/025602 divulgue l'utilisation de matériaux nano-poreux - et notamment de silice nano-poreuse - pour le fractionnement, la stabilisation et le stockage de biomolécules.

US2011213270 décrit un dispositif selon le préambule de la revendication 1. Un procédé de prélèvement d'espèces biologiques ou biochimiques peut comporter les étapes suivantes :
a) disposer une surface de capture desdites espèces biologiques ou biochimiques en contact avec un tissu ou fluide biologique, de telle sorte qu'au moins une espèce biologique ou biochimique soit adsorbée par ladite surface ; et
b) rincer ladite surface pour éliminer les espèces biologiques ou biochimiques n'ayant pas été adsorbées ;
   dans lequel ladite étape a) ne présente pas un caractère chirurgical ;
   le procédé étant caractérisé en ce que ladite surface de capture est la surface d'un matériau nano-poreux.

Le tissu biologique peut être notamment un tissu autre qu'un tissu liquide tel que le sang. Il peut s'agir par exemple d'un épithélium, et notamment d'un endothélium, ou d'un tissu conjonctif. Le fluide biologique (généralement un liquide) peut être notamment le sang entier, le plasma sanguin, le liquide céphalo-rachidien, la salive, etc. Il peut s'agir de tissus ou fluides humains, animaux (non humains) et/ou végétaux.

On entend par « matériau nanoporeux » un matériau cristallin ou amorphe, d'un seul tenant et de préférence de composition homogène, présentant des pores dont le diamètre moyen est inférieur à un micromètre et en particulier inférieur ou égal à 100 nm. On distingue notamment, parmi les matériaux nano-poreux, les matériaux microporeux (pores de diamètre moyen compris entre 0,2 et 2 nm), mésoporeux (pores de diamètre moyen compris entre 2 et 50 nm) et macroporeux (pores de diamètre moyen compris entre 50 et 1000 nm). De préférence la porosité (rapport du volume des pores au volume total) sera supérieure ou égale à 10%.

Les espèces biologiques adsorbées peuvent être des cellules (diamètre compris entre 1 µm et 50 µm environ), des bactéries, des virus, des vésicules circulantes comme les exosomes (diamètre compris entre 20 et 200 nm environ). Les espèces biochimiques adsorbées peuvent être molécules ou des macromolécules, telles que des protéines (diamètre compris entre quelques nanomètres et quelques dizaines de nanomètres), des peptides (taille de l'ordre du nanomètre), des métabolites. La « taille » de ces molécules s'entend comme leur plus grande dimension.

On considère qu'une opération ne présente pas un caractère chirurgical lorsqu'elle n'est pas intrusive, ou en tout cas lorsqu'elle ne représente pas une intervention physique substantielle sur le corps, ne nécessite d'une expertise médicale professionnelle et n'entraine pas de risque substantiel pour la santé. D'une manière générale, ne présente pas un caractère chirurgical toute opération consistant à mettre en contact la surface de capture avec l'épiderme, un tissu accessible par des passages ou ouvertures naturels (rectum, intérieur de la bouche, urètre, vessie...), ou bien par une simple pénétration de l'épiderme, voire par un cathéter intraveineux. Ne présente pas non plus un caractère chirurgical la mise en contact de la surface de capture avec un tissu rendu accessible par une opération chirurgicale préalable (par exemple, introduction d'un cathéter, lorsque cette opération est de nature chirurgicale) ou concomitante, mais réalisée de manière indépendante.

Le procédé peut comporter également une étape c) consistant à analyser les espèces biologiques ou biochimique adsorbées par ladite surface de capture, cette dernière étant utilisée comme support d'analyse. En particulier, ladite étape c) peut être mise en oeuvre par une méthode choisie parmi la spectroscopie de masse avec désorption laser et un procédé d'imagerie tel que l'imagerie de fluorescence. Notamment, ladite étape c) peut être mise en oeuvre par une méthode de spectroscopie de masse avec désorption laser de type MALDI (désorption/ionisation laser assistée par matrice) ou SELDI (désorption/ionisation laser amplifiée par une surface), une matrice organique étant déposée directement sur la surface de capture après ladite étape b) de rinçage. En variante, l'étape c) peut également être mise en oeuvre par une méthode de spectrométrie de diffusion Raman.

Le fait que le matériau de capture puisse servir comme support d'analyse constitue une caractéristique particulièrement avantageuse de l'invention. En effet, le transfert de la surface de capture vers un support d'analyse distinct pourrait abîmer les espèces biochimiques ou biologiques capturées, qui sont souvent très fragiles. En outre ce transfert serait une source de complexité, de coûts et de risques de contamination ou d'erreur. Par comparaison, dans le cas du document WO 2011/025602 précité, une étape d'élution est nécessaire pour pouvoir analyser les molécules capturées et stabilisées.

Ledit matériau nano-poreux peut être du silicium nano-poreux. Avantageusement, ledit silicium nano-poreux peut présenter au moins une des propriétés suivantes (et préférentiellement l'ensemble de ces propriétés) :
- des pores de structure dendritique ;
- des pores de diamètre moyen compris entre 1 et 100 nm ; et
- une porosité comprise entre 40% et 65% sur une profondeur comprise entre 10 nm et 100 µm.

Ladite surface de capture n'est pas fonctionnalisée.

Ladite étape a) peut être effectuée *ex vivo*, sur un échantillon de tissu ou fluide biologique préalablement prélevé d'un organisme humain, animal ou végétal ; de préférence, il pourra s'agir d'un tissu « frais », c'est-à-dire n'ayant pas été soumis à un traitement de congélation ou fixation. En variante, ladite étape a) peut être effectuée *in vivo*, à la condition que cela n'implique pas une intervention physique substantielle sur le corps nécessitant une expertise médicale et comportant un risque substantiel pour la santé d'un patient - c'est-à-dire à la condition que cela n'implique pas une intervention de nature chirurgicale.

Un procédé d'analyse d'espèces biologiques ou biochimiques préalablement adsorbées sur la surface d'un matériau nano-poreux, peut utiliser ladite surface en tant que support d'analyse. En particulier, ladite analyse peut être réalisée par une méthode choisie parmi la spectroscopie de masse avec désorption laser et un procédé d'imagerie tel que l'imagerie de fluorescence. Notamment, ladite analyse peut être réalisée par une méthode de spectroscopie de masse avec désorption laser de type MALDI (désorption/ionisation laser assistée par matrice) ou SELDI (désorption/ionisation laser amplifiée par une surface), à l'aide d'une matrice organique déposée directement sur la surface du matériau nano-poreux. Il peut également s'agir de méthodes d'imagerie, telle l'imagerie de fluorescence ou la colorimétrie, ces analyses pouvant être précédées par l'ajout d'un marqueur adéquat dans le milieu. Il peut également s'agir d'une analyse par spectrométrie de diffusion Raman.

Ledit matériau nano-poreux peut être du silicium nano-poreux. Avantageusement, ledit silicium nano-poreux peut présenter au moins une des propriétés suivantes (et préférentiellement l'ensemble de ces propriétés) :
- des pores de structure dendritique ;
- des pores de diamètre moyen compris entre 1 et 100 nm ; et
- une porosité comprise entre 40% et 65% sur une profondeur comprise entre 10 nm et 100 µm.

Ladite surface de capture n'est pas fonctionnalisée.

Un objet de l'invention est un dispositif de prélèvement d'espèces biologiques ou biochimiques comportant une tige à laquelle est fixé un matériau présentant une surface de capture desdites espèces biologiques ou biochimiques, agencée de manière à pouvoir être mise en contact avec un tissu ou fluide biologique, caractérisé en ce que ledit matériau est un matériau nano-poreux selon la revendication 1.

Avantageusement, ledit silicium nano-poreux peut présenter au moins une des propriétés suivantes (et préférentiellement l'ensemble de ces propriétés) :
- des pores de structure dendritique ;
- des pores de diamètre moyen compris entre 1 et 100 nm ; et
- une porosité comprise entre 40% et 65% sur une profondeur comprise entre 10 nm et 100 µm.

De préférence, ladite surface de capture n'est pas fonctionnalisée.

Ladite tige est placée à l'intérieur d'un tube guide présentant une ouverture latérale ou axiale, ladite tige pouvant être déplacée à l'intérieur dudit tube guide de manière à amener ladite surface de capture en correspondance de ladite ouverture. En particulier, ladite tige peut présenter un méplat auquel est fixé ledit matériau et ledit tube guide peut présenter une ouverture latérale, la surface de capture pouvant être amenée en regard de ladite ouverture par rotation de la tige à l'intérieur du tube guide.

D'autres caractéristiques, détails et avantages de l'invention ressortiront à la lecture de la description faite en référence aux dessins annexés, donnés à titre d'exemple, dans lesquels :
- Les figures 1A - 1D illustrent schématiquement un dispositif selon l'invention ;
- Les figures 2A - 2C et 3A, 3B montrent des vues au microscope électronique, respectivement de la tranche et de la surface, de différents échantillons de silicium nano-poreux pouvant convenir pour la mise en oeuvre de l'invention ;
- Les figures 4, 5 et 6 montrent des spectres de masse de protéines obtenus en appliquant un procédé selon un mode de réalisation de l'invention à l'étude : du plasma sanguin humain, du liquide céphalo-rachidien humain et du tissu cérébral de souris, respectivement ;
- La figure 7 montre des images de fluorescence mettant en évidence la capture de cellules d'un tissu cérébral de souris à l'aide d'un dispositif selon l'invention ; et
- La figure 8 illustre un dispositif selon un mode de réalisation de l'invention.

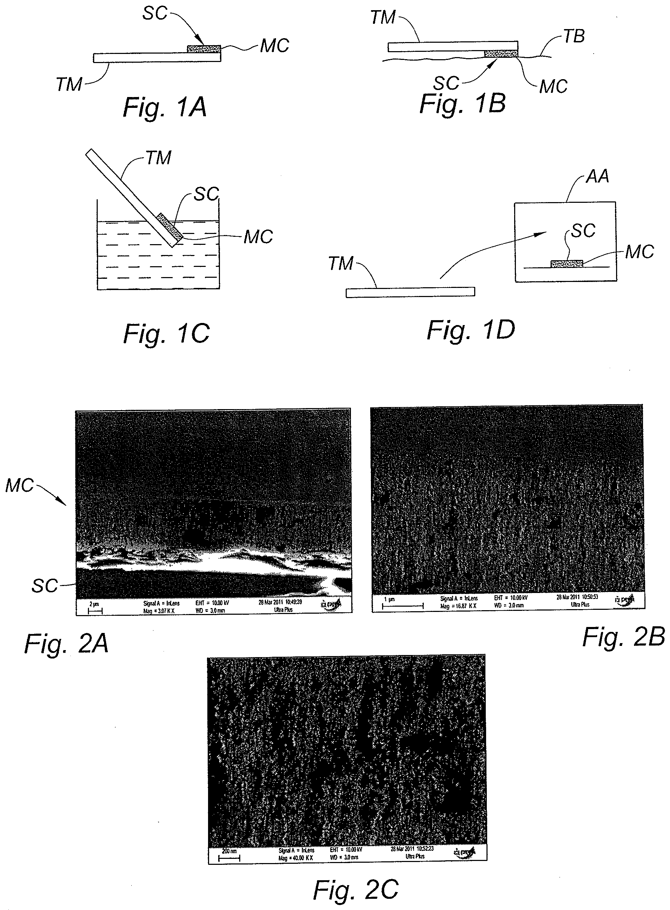

La figure 1A illustre schématiquement un dispositif pas selon un mode de réalisation de l'invention, constitué par une plaquette MC d'un matériau de capture, de type nano-poreux, fixée - de préférence de manière amovible - sur une tige de manipulation TM. La plaquette du matériau de capture présente, à l'opposé de sa surface de fixation à la tige TM, une surface de capture nano-poreuse SC, ayant typiquement une dimension comprise entre 0,5 et 5 cm². Le matériau de capture peut être du silicium nano-poreux, et plus particulièrement mésoporeux.

Comme illustré sur la figure 1 B, la tige TM est utilisée pour amener la surface de capture SC en contact avec un tissu biologique TB. Il peut s'agir d'un échantillon de tissu préalablement prélevé d'un corps humain ou animal, voire d'un organisme végétal (*ex vivo*), ou bien d'un tissu *in vivo*. La mise en contact peut se faire par simple apposition, sans qu'il soit nécessaire de frotter le tissu ou d'appliquer une pression élevée. Cela est important, surtout pour les applications *in vivo*.

En variante, le matériau de capture pourrait être immergé dans un fluide biologique, ou une gouttelette dudit fluide pourrait être déposée sur la surface de capture.

La surface de capture en silicium nano-poreux est lisse au toucher et ne présente pas d'aspérités significative. Ainsi le risque de lésion du tissu est minimisé, ce qui est très avantageux pour les applications *in vivo.* Le prélèvement des espèces biologiques ou biochimiques n'est donc pas réalisé par micro-abrasion, ni par fonctionnalisation chimique, mais par un effet de succion dû aux nano-pores. Cet effet conduit à une fixation préférentielle des peptides et des « petites » protéines, présentant une taille de l'ordre de 1 à 5 nm environ, et/ou une masse comprise entre 5000 et 20.000 Da environ. Cela est avantageux, car ces « petites » protéines sont généralement plus utiles en tant que marqueurs que des molécules de plus grandes dimensions. L'effet de succion explique également l'adhésion des cellules, qui sont trop grandes pour pouvoir pénétrer dans les pores.

Ensuite (figure 1C) le dispositif est éloigné du tissu, et la surface de capture est rincée, par exemple par immersion dans l'eau, ou à l'aide d'une solution tampon, ce qui permet d'éliminer les espèces biologiques ou biochimiques n'ayant pas été adsorbées par la surface, et donc n'adhérant pas forcément à elle. Ce rinçage permet de révéler le caractère sélectif de l'adsorption par la surface nano-poreuse.

Enfin, la plaquette MC de matériau de capture est désolidarisée de la tige (bien que cela ne soit pas toujours nécessaire) et introduite dans un appareillage d'analyse AA, permettant d'analyser les espèces biologiques ou biochimique adsorbées par la surface de capture. Typiquement, les analyses pourront être effectuées par spectroscopie de masse avec désorption laser, de type MALDI ou SELDI. Dans ce cas, il sera nécessaire de déposer sur la surface de capture une matrice organique adaptée. En variante ou en complément, il sera possible d'effectuer des analyses par imagerie de fluorescence. En tout cas, le matériau de capture MC sert directement de support d'analyse. Pour les analyses avec désorption et ionisation laser, il est nécessaire que le matériau de capture soit conducteur - ce qui est le cas du silicium nano-poreux dopé.

Comme cela a été dit plus haut, le matériau de capture utilisé de manière préférentielle est le silicium nano-poreux, même s'il est tout à fait possible d'utiliser d'autres matériaux nano-poreux.

Du silicium nano-poreux (et plus précisément mésoporeux) peut être obtenu par anodisation électrochimique de silicium dopé p+ de conductivité de 10 à 20 mΩ.cm dans une solution d'acide fluorhydrique à environ 15%. Pour ce faire, le matériau est trempé dans un bain d'HF est soumis à une électrolyse, ce procédé étant connu. On obtient de cette façon un matériau présentant une porosité de structure dendritique ; cela signifie que les pores n'ont pas un axe rectiligne ; ils s'étendent dans la profondeur du matériau selon une direction discontinue, et peuvent se croiser. Cette structure dendritique favorise la succion. La porosité (rapport du volume des pores sur le volume total) est comprise entre 40 et 65%. La porosité s'étend sur une profondeur d'environ 6µm. Au-delà, on retrouve le silicium massif. D'une façon générale, dans cette invention, la porosité du matériau peut s'étendre sur une profondeur pouvant être comprise entre 10 nm et 100 µm.

En faisant varier les paramètres de fabrication (concentration en HF, temps d'anodisation, densité de courant, type de silicium) on peut faire varier ces caractéristiques aisément.

En variante, il est possible de réaliser du silicium nano-poreux présentant une structure ordonnée au moyen d'un processus de lithographie électronique.

Les figures 2A, 2B et 2C montrent des images de microscopie électronique de la tranche d'un échantillon de silicium nano-poreux obtenu par le procédé d'anodisation électrochimique décrit ci-dessus. Les figures présentent des grossissements différents ; en particulier, la figure 2B met en évidence la transition nette entre silicium massif et silicium nano-poreux, tandis que la figure 2C permet d'apprécier la structure dendritique des pores.

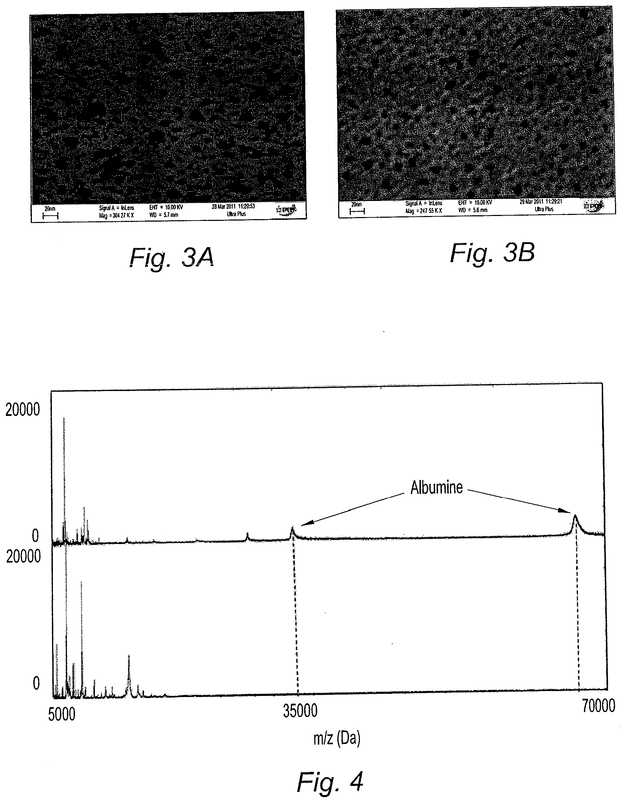

Les figures 3A et 3B montrent des images de microscopie électronique de la surface de deux échantillons de silicium nano-poreux obtenus par anodisation électrochimique dans des conditions différentes.

Le dispositif de l'invention a été testé au moyen de trois essais *ex vivo.*

Dans un premier essai, 5 µl de plasma sanguin humain ont été déposés directement sur la surface de capture en silicium nano-poreux. Ensuite, la surface a été rincée deux fois à l'aide d'un tampon acide (Acétate de Sodium 100mM, pH 4,0) pendant 1 min. Le silicium présentant une charge de surface légèrement chargée négativement, on utilise une solution tampon dont le pH est ajusté pour favoriser une charge positive des protéines de l'échantillon que l'on souhaite collecter. Autrement dit, le pH est ajusté en fonction du point isoélectrique des protéines d'intérêt. On rappelle que le point isoélectrique d'une protéine correspond à la valeur de pH pour laquelle la charge électrique de ladite protéine est nulle. Ce rinçage permet d'éliminer les espèces n'ayant pas adhéré à la surface de capture, ou des impuretés (résidus de tissu, sang...). Ensuite, la surface a été rincée encore une fois dans l'eau, puis elle a été séchée à l'air libre. Une matrice organique (acide sinapinique) a été déposée sur la surface de capture, qui a ensuite été soumise à une analyse MALDI au moyen d'un spectromètre de masse MALDI du commerce (Bruker Autoflex). L'acquisition automatique des spectres a été réalisée sur 5400 impacts laser répartis régulièrement pour chaque échantillon. L'analyse a été effectuée en mode linéaire (ce qui signifie que les protéines décrivent une trajectoire linéaire dans le spectromètre de masse) avec une intensité de 55 (réglage de l'appareil), avec une atténuation du signal de matrice à 1000 Da.

Les résultats sont représentés sur la figure 4, où m/z est le rapport masse/charge (en Dalton/unités élémentaires de charge) et l'axe des abscisses représente l'intensité du signal de spectroscopie de masse (unités arbitraires). Le graphique inférieur correspond à l'utilisation du matériau nano-poreux, conformément à l'invention ; le graphique supérieur sert de référence et a été obtenu en utilisant un porte-substrat du type normalement utilisé en spectrométrie de masse (barrette métallique lisse sur laquelle est déposé un polymère), dont la référence est Biorad CM10. Les mesures sur le support de référence ont été réalisées selon le même protocole que celles sur la surface de silicium nano-poreux.

On observe que les espèces caractérisées par un rapport m/z>10000 - et notamment l'albumine, constituant abondant et peu intéressant pour établir un diagnostic - ne sont pas détectées lorsqu'on utilise une surface de capture en silicium nano-poreux, ce qui démontre la sélectivité de la capture. Inversement, on observe un enrichissement des pics correspondant aux protéines de faible masse ; dans le cas du support lisse, au contraire, ces protéines sont en grande partie éliminées par le rinçage.

On observe également que les spectres sont plus riches sur les pics correspondant à des protéines de faible masse, ce qui confirme une bonne adhésion des petites protéines sur la surface nano-poreuse, en dépit des opérations de rinçages. Sur le support de contrôle, lisse, les petites protéines sont en grande partie éliminées par le rinçage.

Dans un deuxième essai, 10 µl de liquide céphalo-rachidien humain ont été déposés directement sur la surface de capture en silicium nano-poreux, du même type que celle utilisée pour le premier essai. Ensuite, la surface a été rincée deux fois à l'aide d'un tampon acide (Acétate de Sodium 100mM, pH 4,0) pendant 1 min. Ce rinçage permet d'éliminer les espèces n'ayant pas adhéré à la surface de capture, ou des impuretés (résidus de tissu, sang...). Ensuite, la surface a été rincée encore une fois dans l'eau, puis elle a été séchée à l'air libre. Une matrice organique (acide sinapinique pour l'analyse des protéines, CHCA, c'est-à-dire acide α-cyano-4-hydroxycinnamique, pour l'analyse des peptides) a été déposée sur la surface de capture, qui a ensuite été soumise à une analyse au moyen d'un spectromètre de masse SELDI du commerce (Biorad PCS 4000).

Les paramètres de lecture ont été réglés en fonction de l'échelle de masse des espèces à détecter. Les conditions optimales ont été déterminées manuellement sur quelques spots avant de lancer l'acquisition automatique sur 583 impacts laser répartis régulièrement pour chaque échantillon :
Pour les peptides (bas poids moléculaires), on a utilisé une intensité de 1000 nJ et une atténuation du signal de matrice à 500 Da.

Pour les protéines (hauts poids moléculaires) on a utilisé une intensité de 2200 nJ et une atténuation du signal de matrice à 1000 Da.

Comme dans le cas du premier essai, la même analyse a été effectuée également en utilisant un porte-substrat lisse Biorad CM10.

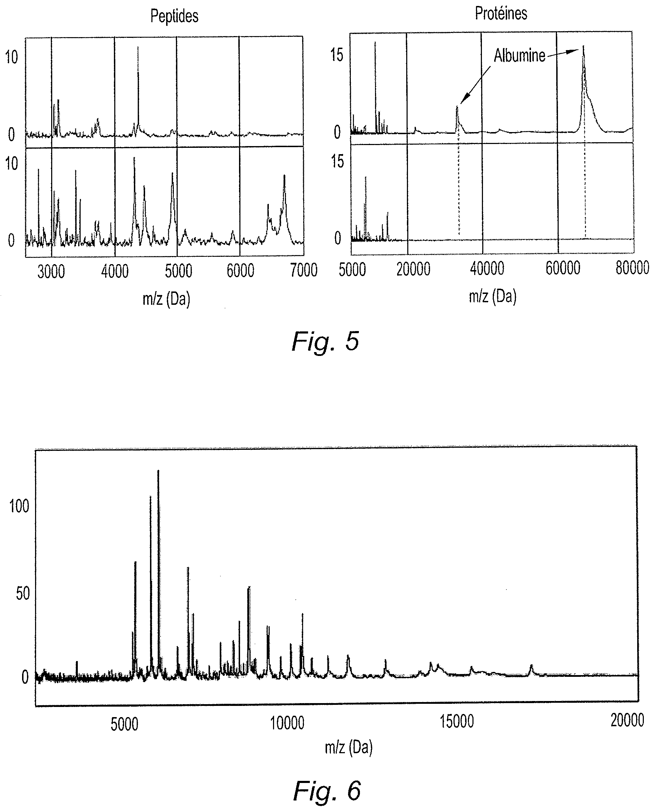

Les résultats sont représentés sur la figure 5, où les graphiques supérieurs ont été obtenus avec le substrat lisse de référence et les graphiques supérieurs ont été obtenus avec une surface de capture nano-poreuse, conformément à l'invention. Concernant les protéines, on confirme les résultats du premier essai : les « petites » protéines sont fixées efficacement tandis qu'on observe une élimination substantielle des « grandes » protéines, et notamment de l'albumine. La différence entre les deux supports est encore plus considérable dans le cas des peptides : ces derniers sont largement éliminés par rinçage dans le cas du support lisse, alors que dans le cas du support nano-poreux on observe un spectre très riche. Les mesures sur le support de référence ont été réalisées selon le même protocole que celles sur la surface de silicium nano-poreux.

Dans un troisième essai, un échantillon de tissu cérébral frais de souris a été apposé sur la surface de capture en silicium nano-poreux, du même type que celle utilisée pour le premier et le deuxième essai. Ensuite, la surface a été rincée deux fois à l'aide d'un tampon acide (Acétate de Sodium 100mM, pH 4,0) pendant 1 min. Ce rinçage permet d'éliminer les espèces n'ayant pas adhéré à la surface de capture, ou des impuretés (résidus de tissu, sang...). Ensuite, la surface a été rincée encore une fois dans l'eau, puis elle a été séchée à l'air libre.

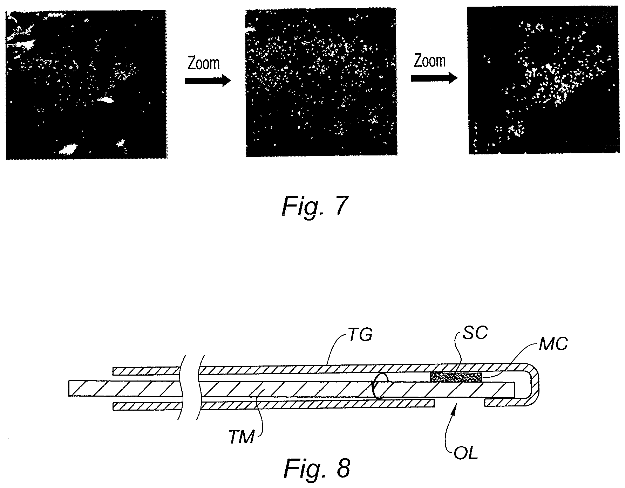

Les cellules capturées ont été mises en évidence par imagerie de fluorescence, grâce à l'ajout d'un intercalant de l'ADN (tampon Hoechst) - voir figure 7. Cette figure révèle la présence de cellules sur la surface de capture après rinçage.

Une matrice organique (acide sinapinique) a été déposée sur la surface de capture, qui a ensuite été soumise à une analyse au moyen d'un spectromètre de masse SELDI du commerce (Biorad PCS 4000). Les résultats sont représentés sur la figure 4, où m/z est le rapport masse/charge (en Dalton/unités élémentaires de charge) et l'axe des abscisses représente l'intensité du signal de spectroscopie de masse (unités arbitraires). Encore une fois, on observe un spectre de masse très riche dans la région 5000 - 10000 Da.

La figure 8 (qui n'est pas à l'échelle) illustre un mode de réalisation d'un dispositif de l'invention qui est particulièrement adapté aux applications in vivo.

Dans ce dispositif, la tige TM est flexible et présente un diamètre de 500 µm et une longueur de 20 cm. A environ 1 cm de son extrémité se trouve un méplat long 2 cm, où est fixé, de manière amovible, le matériau de capture MC. La tige coulisse dans un tube guide ou cathéter TG en matériau biocompatible, présentant un diamètre externe de 1 mm et une paroi de 50 µm d'épaisseur. L'extrémité distale du tube est fermée ; à une distance d'environ 1 cm de cette dernière, une ouverture latérale OL est ménagée sur une longueur de 2 cm. Initialement, comme illustré par la figure, la tige est agencée de telle manière que le la surface de capture SC soit écartée de l'ouverture. Le tube guide avec la tige est introduit dans le corps d'un patient, jusqu'à ce que l'ouverture OL se trouve en regard du tissu à analyser. Une rotation de 180° de la tige autour de son axe à l'intérieur du tube permet d'amener la surface de capture SC en correspondance de ladite ouverture, et donc en contact avec le tissu. Une autre rotation de 180° amène la surface de capture dans sa position initiale. Ensuite, l'ensemble tube-guide est extrait du corps du patient, le matériau de capture est désolidarisé de la tige et soumis aux étapes de rinçage et analyse comme décrit ci-dessus. En variante, le tube guide peut avoir été introduit préalablement dans le corps du patient ; dans ce cas, il est seulement nécessaire d'introduire la tige dans le tube, effectuer la double rotation et l'extraire.

En variante, le tube peut présenter une ouverture axiale, au niveau de son extrémité distale. Dans ce cas, la surface de capture est amenée en contact avec le tissu par un mouvement d'avancée de la tige.

Il est entendu que d'autres dispositifs selon la revendication 1 peuvent être utilisés pour réaliser une analyse *in vivo* conformément à l'invention.

## Revendications

1. Dispositif de prélèvement in vivo d'espèces biologiques ou biochimiques comportant une tige (TM) à laquelle est fixé un matériau (MC) nano-poreux présentant une surface de capture (SC) desdites espèces biologiques ou biochimiques, agencée de manière à pouvoir être mise en contact avec un tissu (TB) ou fluide biologique, ladite tige est placée à l'intérieur d'un tube guide (TG) **caractérisé en ce que** le tube guide présente une ouverture latérale (OL) ou axiale, ladite tige pouvant être déplacée à l'intérieur dudit tube guide de manière à amener ladite surface de capture en correspondance de ladite ouverture.

2. Dispositif selon la revendication 1, dans lequel ledit matériau nano-poreux est du silicium nano-poreux, de préférence du silicium nano-poreux présentant au moins une des propriétés suivantes :
- des pores de structure dendritique ;
- des pores de diamètre moyen compris entre 1 et 100 nm ; et
- une porosité comprise entre 40% et 65% sur une profondeur comprise entre 10 nm et 100 µm.

3. Dispositif selon l'une des revendications 1 ou 2, dans lequel ladite surface de capture n'est pas fonctionnalisée.

4. Dispositif selon l'une des revendications 1 à 3 dans lequel ladite tige (TM) présente un méplat auquel est fixé ledit matériau (MC), la surface de capture (SC) pouvant être amenée en regard de ladite ouverture par rotation ou par un mouvement d'avancée de la tige à l'intérieur du tube guide.

## Patentansprüche

1. Vorrichtung zur in vivo-Probennahme von biologischen oder biochemischen Spezies, aufweisend einen Stab (TM), an dem ein nanoporöses Material (MC) befestigt ist, das eine Einfangfläche (SC) der biologischen oder biochemischen Spezies aufweist, die derart ausgebildet ist, dass sie mit einem biologischen Gewebe (TB) oder Fluid in Kontakt versetzbar ist, wobei der Stab in einem Führungsrohr (TG) platziert ist, **dadurch gekennzeichnet, dass** das Führungsrohr eine seitliche oder axiale Öffnung (OL) aufweist, wobei der Stab innerhalb des Führungsrohrs derart verlagerbar ist, dass die Einfangfläche in Übereinstimmung mit der Öffnung geführt wird.

2. Vorrichtung nach Anspruch 1, wobei das nanoporöse Material nanoporöses Silizium ist, vorzugsweise nanoporöses Silizium, das mindestens eine der folgenden Eigenschaften aufweist:
- Poren mit dendritischer Struktur,
- Poren mit einem durchschnittlichen Durchmesser zwischen 1 und 100 nm inklusive,
- eine Porosität zwischen 40 % und 65 % inklusive über eine Tiefe zwischen 10 nm und 100 µm inklusive.

3. Vorrichtung nach einem der Ansprüche 1 oder 2, wobei die Einfangfläche nicht funktionalisiert ist.

4. Vorrichtung nach einem der Ansprüche 1 bis 3, wobei der Stab (TM) eine Abflachung aufweist, an der das Material (MC) befestigt ist, wobei die Einfangfläche (SC) mittels Rotation oder einer Vorschubbewegung des Stabs innerhalb des Führungsrohrs gegenüber der Öffnung führbar ist.

## Claims

1. A device for sampling in vivo biological or biochemical species comprising a rod (MR) to which a nanoporous material (CM) is fixed having a capture surface (CS) of said biological or biochemical species, arranged so that it can be brought into contact with a biological tissue (BT) or fluid, said rod is placed inside a guide tube (GT) **characterized in that** the tube has a lateral (OL) or axial opening, said rod being movable within said guide tube so as to bring said capture surface into correspondence with said opening.

2. The device as claimed in claim 1, wherein said nanoporous material is nanoporous silicon, preferably nanoporous silicon having at least one of the following properties:
- pores of dendritic structure;
- pores with an average diameter between 1 and 100 nm; and
- a porosity between 40% and 65% to a depth between 10 nm and 100 µm.

3. The device as claimed in claim 1 or 2, wherein said capture surface is not functionalized.

4. The device as claimed in one of claims 1 to 3, wherein said rod (TM) has a flat surface to which said material (CM) is fixed, the capture surface (CS) can be brought opposite said opening by rotation or by a movement of advancement of the rod within the guide tube.
